# EUROPEAN PATENT APPLICATION

(11) **EP 4 670 691 A1**
(43) Date of publication of application: **31.12.2025**
(21) Application number: 25197237.8
(22) Date of filing: 21.08.2025
(51) Int. Cl.: A61F 11/08, H04R 1/10, H04R 25/00

(54) **EARPLUG INTERCHANGEABLE FACEPLATE**

(30) Priority: 25.06.2024 US 202418752999
(71) Applicant: Decibullz LLC, Fort Collins, CO 80525 (US)
(72) Inventor: KIRKPATRICK, Kyle J, Fort Collins, 80528 (US)
(74) Representative: Kador & Partner Part mbB

(57) **Abstract**

A faceplate (1) configured to be removably affixed to an outer face (20) of a retaining body (17) of an earpiece (2) configured to be disposed within the concha of an ear of a wearer.
An earpiece (2) comprising a retaining body (17) configured to be disposed within the concha of an ear of a wearer, the retaining body having an outer face (20) opposite to an inner face (21) extending to a retaining body periphery (22), the retaining body including a projection element (18) configured to project into an ear canal opening of the wearer, the projection element (18) adapted to retain an earpiece tip (19) configured to engage an ear canal wall of the wearer, and a faceplate (1) configured to cover the outer face (20) of the retaining body (17), the retaining body and the faceplate including releasably engageable mateable parts to removably affix the faceplate to the retaining body.

## Description

### I. FIELD OF THE INVENTION

A faceplate configured to removably affix to an outer face of an earpiece configured to dispose within the concha of an ear of a wearer.

### II. BACKGROUND OF THE INVENTION

Conventional earpieces or earplugs come in a numerous and wide variety of structure and function. However, conventional earpieces or earplugs once obtained by the wearer may not allow the structure or function to be substantially changed or repeatedly changed to customize or personalize the look and the feel of the earpiece or earplug.

Accordingly, there would be a substantial advantage in providing an earpiece and a method of making an earpiece and a method of using an earpiece that allows the wearer to repeatedly change the outward appearance of the earpiece or earplug to customize or personalize the look and feel of the earpiece.

### III. SUMMARY OF THE INVENTION

Accordingly, a broad object of particular embodiments of the invention can be to provide a faceplate configured to removably affix to an outer face of an earpiece, the earpiece configured to dispose within the concha of an ear of a wearer.

Another broad object of particular embodiments of the invention can be to provide an earpiece including a retaining body configured to dispose within the concha of an ear of a wearer, the retaining body including a retaining body outer face opposite a retaining body inner face extending to a retaining body periphery, wherein the retaining body inner face including a projection element configured to project into an ear canal opening of the wearer upon disposing the retaining body within the concha, the projection element adapted to retain an earpiece tip configured to engage an ear canal wall of the wearer, and a faceplate configured to cover the retaining body outer face of the retaining body, wherein the retaining body and the faceplate including releasably engageable mateable parts to removably affix the faceplate to the retaining body.

Another broad object of the invention can be to provision of a method of making an earpiece including configuring a retaining body to dispose within the concha of an ear of a wearer, the retaining body having a retaining body outer face opposite a retaining body inner face extending to a retaining body periphery, the retaining body inner face including a projection element configured to project into an ear canal opening of the wearer upon disposing the retaining body within the concha, the projection element adapted to retain an earpiece tip configured to engage an ear canal wall of the wearer; and configuring a faceplate to cover the retaining body outer face of the retaining body, the retaining body and the faceplate including releasably engageable mateable parts to removably affix the faceplate to the retaining body.

Another broad object of the invention can be to provision of a method of using an earpiece including disposing a retaining body within the concha of an ear of a wearer, the retaining body having a retaining body outer face opposite a retaining body inner face extending to a retaining body periphery, the retaining body inner face including a projection element configured to project into an ear canal opening of the wearer upon disposing the retaining body within the concha, the projection element adapted to retain an earpiece tip configured to engage an ear canal wall of the wearer; and releasably engaging mateable parts of a faceplate and the retaining body to removably affix the faceplate to the retaining body, the faceplate configured to cover the retaining body outer face of the retaining body.

Naturally, further objects of the invention are disclosed throughout other areas of the specification, drawings, photographs, and claims.

### IV. BRIEF DESCRIPTION OF THE DRAWINGS

Figure 1 is an illustration of a method of using a particular embodiment of the earplug interchangeable faceplate.
Figure 2 is an illustration of the anatomy of the human ear.
Figure 3 is a front perspective view of a pair of earplugs with the left earplug including an embodiment of the interchangeable faceplate coupled to the intra-auricular support and an exploded view of the right earplug depicting an embodiment of the interchangeable faceplate, the retaining body and a tip.
Figure 4 is a rear perspective view of a pair of earplugs with the left earplug including an embodiment of the interchangeable faceplate coupled to the retaining body and an exploded view of the right earplug depicting an embodiment of the interchangeable faceplate, the retaining body and the tip.
Figure 5A is a rear perspective view of an embodiment of a left earplug including an embodiment of the interchangeable faceplate coupled to the intra-auricular support.
Figure 5B is an exploded view of embodiment of the left earplug shown in Figure 5A.
Figure 5C is an exploded side elevation view of the left earplug shown in Figure 5B.
Figure 5D is a rear perspective view of an embodiment of a right earplug including an embodiment of the interchangeable faceplate coupled to the retaining body.
Figure 5E is an exploded view of embodiment of the right earplug shown in Figure 5D.
Figure 5F is an exploded side elevation view of the left earplug shown in Figure 5E.
Figure 6A is a rear perspective view of a pair of earplugs including a left earplug and a right earplug each including an embodiment of the interchangeable faceplate coupled to the retaining body.
Figure 6B is a rear elevation view of the pair of earplugs shown in Figure 5A.
Figure 6C is a front elevation view of the pair of earplugs shown in Figure 5A.
Figure 6D is a top plan view of the pair of earplugs shown in Figure 5A.
Figure 6E is bottom plan view of the pair of earplugs shown in Figure 5A.
Figure 6F is a side elevation view of the pair of earplugs shown in Figure 5A.

### V. DETAILED DESCRIPTION OF THE INVENTION

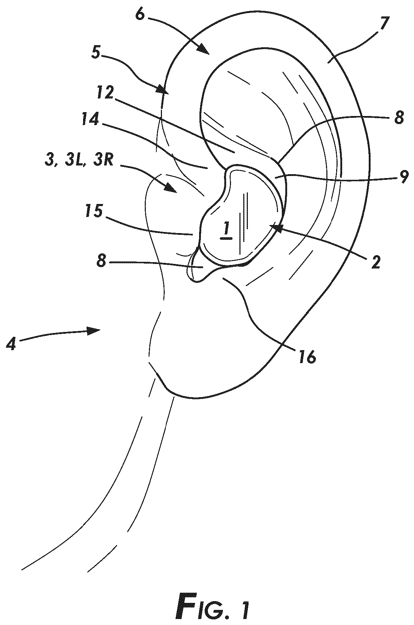

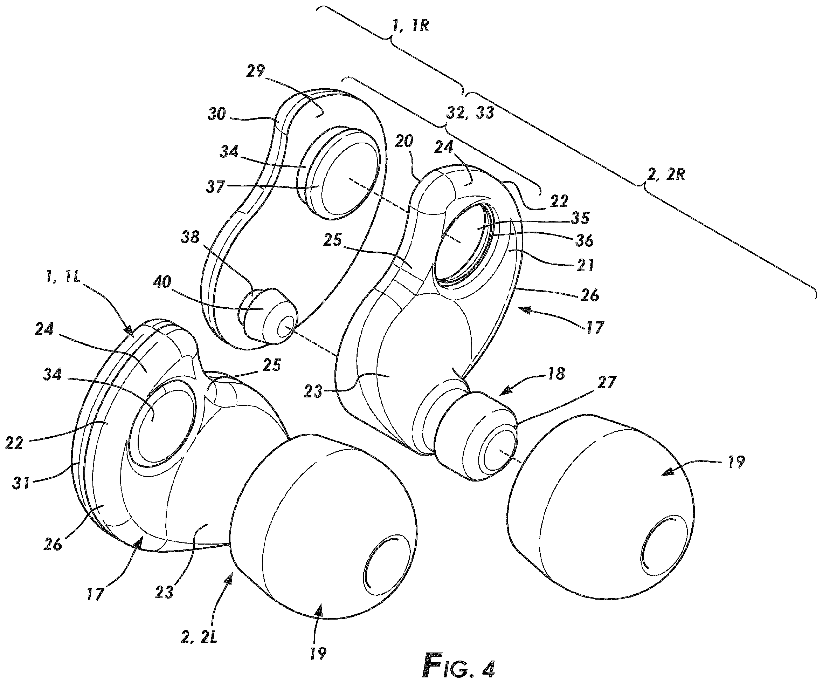

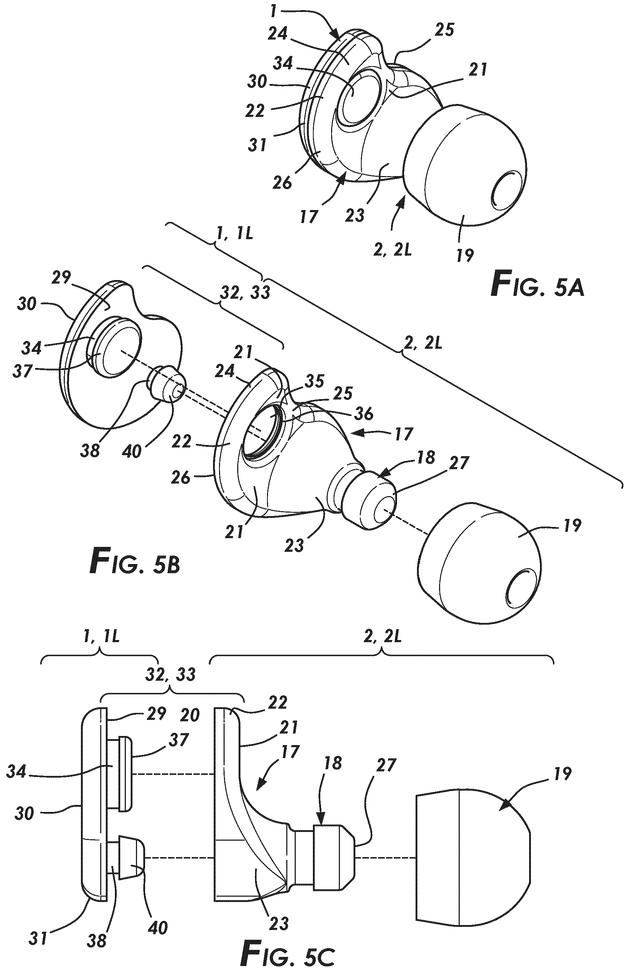

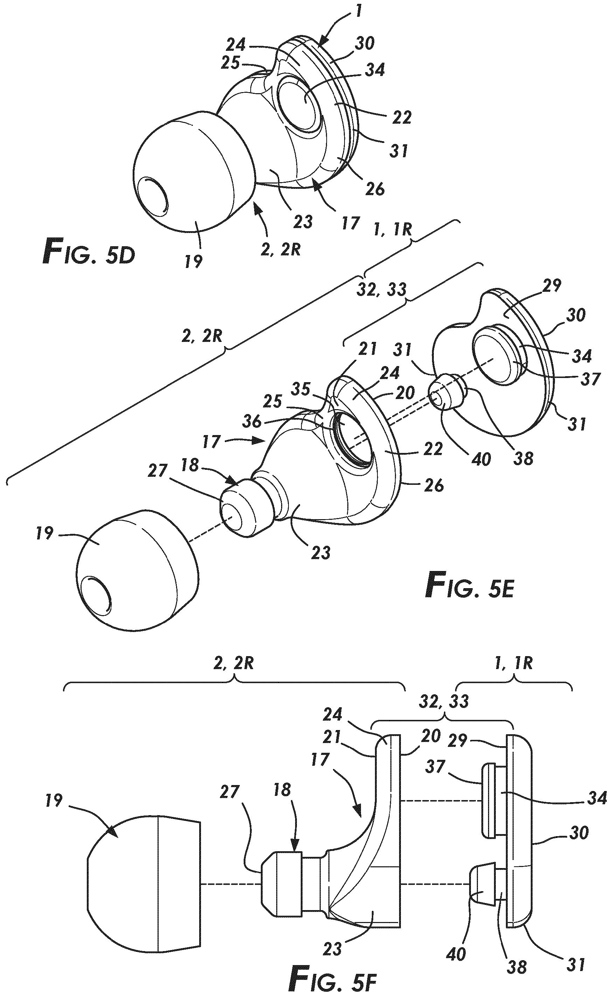

Now referring generally to Figures 1 through 6F, which illustrate a faceplate (1) removably affixable to an earpiece (2) and methods of making the faceplate (1) affixable to the earpiece (2), and a method of using the faceplate (1) removably affixable to the earpiece (2).

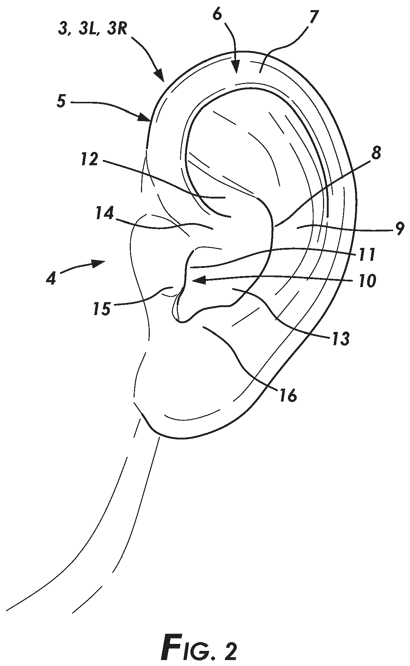

Now, with primary reference to Figure 1, which depicts an embodiment of the faceplate (1) affixed to an earpiece (2) worn in an ear (3) by a wearer (4). Now, with primary reference to Figure 2, for illustrative purposes and to aid in the understanding of the placement of the earpiece (2), an exterior view of an illustrative human ear (3, left ear "3L", right ear "3R"). The external ear (5) is an irregularly concave cartilaginous member including a number of eminences and depressions which give each ear (3, 3L, 3R) a distinct size and/or shape. The auricle (6) of the ear (3), also known as the pinna, is the visible portion of the ear (3). The helix (7) is the curved outer rim of the ear (3). Below the helix (7) is the antihelix (8), a prominence which describes a curve around the concha (9), a relatively deep cavity containing the ear canal opening (10) which provides entry to the ear canal (11) (also referred to as the "auditory canal"). The concha (9) is divided into two parts, the cymba concha (12) and the cavum concha (13), by the crus helix (14) which curves around the outside of the ear (3), and extends inwards at about the vertical midpoint of the ear (3). The cymba concha (12) lies above the crus helix (14) and below the antihelix (8). The cavum concha (13) lies below the crus helix (14) and surrounds the ear canal opening (10). In front of the cavum concha (13) and projecting backwards from the front of the ear (3, 3L, 3R) is the tragus (15), a small semicircular prominence. Opposite the tragus (15) and separated from it by the deep curvature of the intertragic notch is the antitragus (16). Of note, different ears (3) have different sizes and shapes. In this regard, the precise structure of the human ear (3) varies from individual to individual. For example, some ears (3) may have additional features that are not shown in Figures 1 and 2, and some ears (3) may lack some of the features that are shown in Figures 1 and 2. Likewise, some features of different ears (3) may be more or less prominent than those shown in Figures 1 and 2.

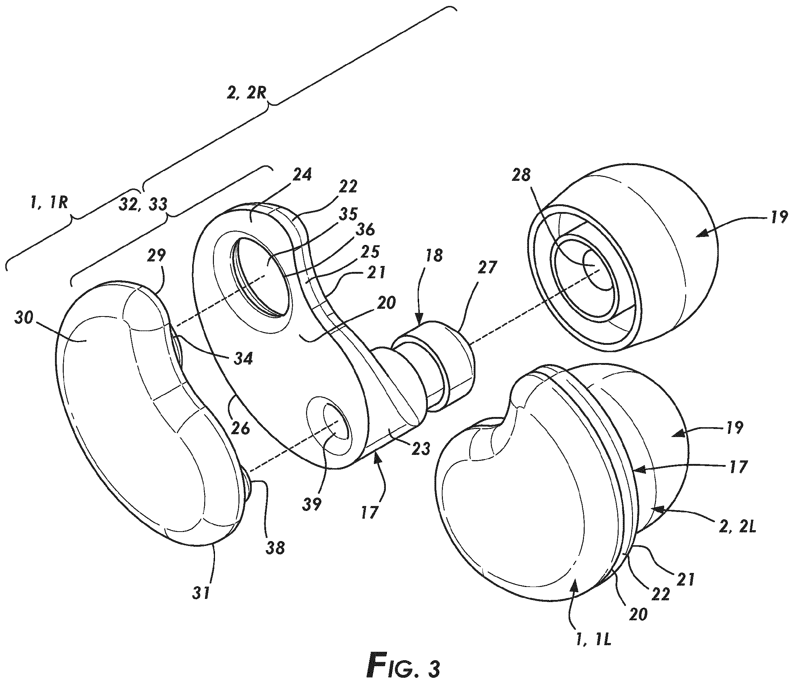

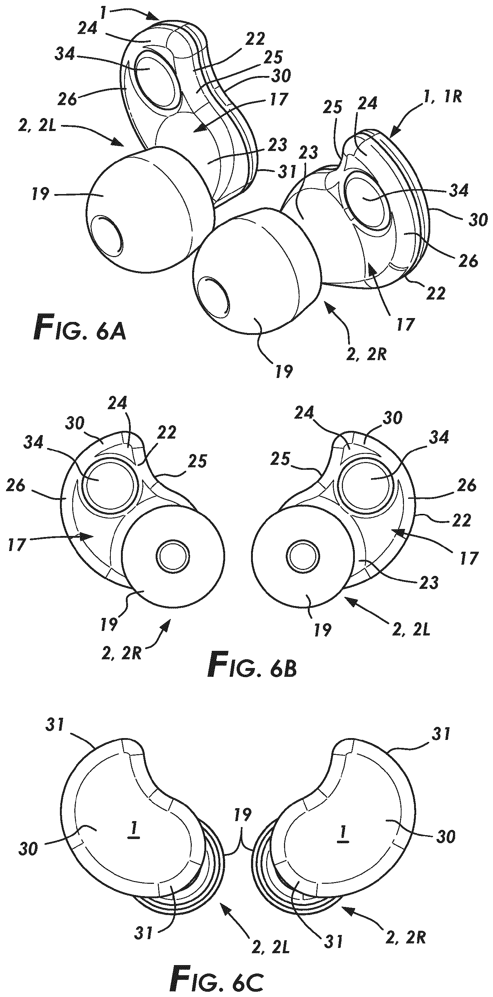

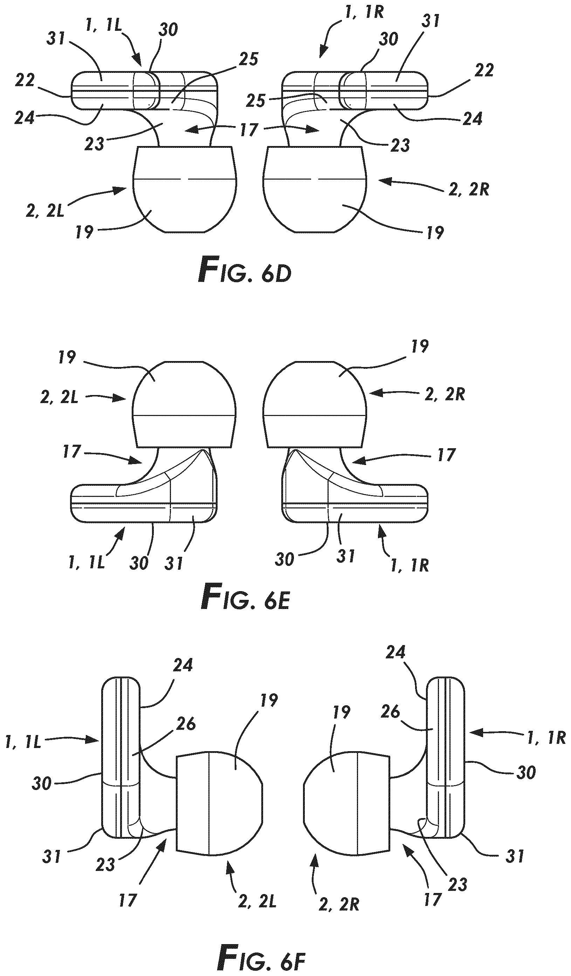

Now, with primary reference to Figures 3 through 4, embodiments of an earpiece (2) suitable for use with embodiments of the removably affixable faceplate (1) can comprise one earpiece (2) configured to be worn in a left ear (3L) or configured to be worn in a right ear (3R) of a wearer (4) or comprise a pair of earpieces (2L, 2R) (also shown by the examples of Figures 6A through 6F), whereby one earpiece (2L) of the pair of earpieces (2L, 2R) can be a mirror image of the other earpiece (2R) of the pair of earpieces (2L, 2R) and the pair of earpieces (2L, 2R) can correspondingly fit in the left ear (3L) (as shown in the example of Figure 1) and right ear (3R) of a wearer (4). Each of the pair of earpieces (2L, 2R) can have substantially the same structure and function, or the same structure and function, including one or more of: a retaining body (17), a projection element (18) outwardly extending therefrom, and an earpiece tip (19) affixable to the projection element (18).

Again, with primary reference to Figures 3 through 4, embodiments of the retaining body (17) of the earpiece (2) can be configured to position within and engage the external ear (5) to maintain the earpiece (2) in the ear (3) of the wearer (4). As shown by the illustrative example or Figure 1, particular embodiments, of the retaining body (17) can be positioned within the area of the external ear (5) anatomically defined by one or more of the concha (9) (also referred to as the "concha bowl"), antihelix (8), antitragus (16), or tragus (15), whereby the retaining body (17) can be of sufficient area to preclude ingress into the ear canal opening (10). As to particular embodiments, the retaining body (17) can be configured to be positioned within the concha (9) concurrent with disposition of the projection element (18) within the ear canal opening (10).

Again, with primary reference to Figures 3 through 4, the retaining body (17) can have a retaining body outer face (20) opposite a retaining body inner face (21) each extending to a retaining body periphery (22). The retaining body inner face (21) can be disposed toward the ear canal opening (10) when the earpiece (2) is worn by the wearer (4); thus, the retaining body inner face (21) can contact a portion of the concha (9) of the ear (3). Correspondingly, the opposing retaining body outer face (20) can be directed away from the ear canal opening (10) when the earpiece (3) is worn by the wearer (4); hence, the retaining body outer face (20) can be visible when the earpiece (2) is worn by the wearer (4). As to particular embodiments, the retaining body inner face (21) excluding the area associated with the projection element (18) can be generally flat. As to particular embodiments, the retaining body outer face (20) can be generally flat. The term "generally flat" means generally level and smooth within normal manufacturing variance. As to particular embodiments, the retaining body inner face (21) and retaining body outer face (20) can be disposed in opposed relation and in particular embodiments substantially parallel opposed relation.

Again, with primary reference to Figures 3 through 4, in particular embodiments, the retaining body (17) can include a first portion (23) which disposes proximate the ear canal opening (10) when the earpiece (2) is worn by a wearer (4), henceforth termed the "proximal portion" (23), generally opposite a second portion (24) which disposes distal from the ear canal opening (10) when the earpiece (2) is worn by a wearer (4), henceforth termed the "distal portion" (24). The proximal portion (23) and distal portion (24) can be connected by a pair of arcuate portions (25, 26), namely an inner arcuate portion (25) and an opposing outer arcuate portion (26), whereby the outer arcuate portion (26) can dispose proximate the antihelix (8) when the earpiece (2) is worn by a wearer (4). Correspondingly, the outer arcuate portion (26) can be curved to generally follow the curve of the antihelix (8) and/or the rear of the cavum concha (13). The retaining body (17) can be configured with a continuous retaining body periphery (22), thereby the portions (23)(24)(25)(26) can all be interconnected or connected to one another to form an integrated or one-piece retaining body (17); however, this particular example is not intended to preclude embodiments having proximal and distal portions that are connected without arcuate portions or by only an inner arcuate portion (25) or only an outer arcuate portion (26), or where the arcuate portions (25, 26) define greater or lesser curvature. This illustrative example of an earpiece (2) is not intended to preclude other configurations of the earpiece (2), but rather is intended to provide examples sufficient for a person of ordinary skill to make and use an earpiece (2) suitable for use with various embodiments of the invention.

Now, with primary reference to Figure 1, the proximal portion (23) of the retaining body (17) can dispose proximate the ear canal opening (10) when the earpiece (2) is worn by a wearer (4). The proximal portion (23) can be convexly arcuate, and can have one or more radii of curvature, which as an illustrative example, can be in a range of between about 4.3 mm and about 8.7 mm. As but one illustrative example, the radius of curvature or the average radius of curvature of the proximal portion (19) can be about 6.5 mm.

The distal portion (24) of the retaining body (17) can dispose distal from the ear canal opening (10) when the earpiece (2) is worn by a wearer (4), whereby the distal portion (24) can be generally opposite the proximal portion (23). The distal portion (24) can be convexly arcuate, and can have one or more radii of curvature, which as an illustrative example, can be in a range of between about 1.3 mm and about 3.3 mm. As but one illustrative example, the radius of curvature or the average radius of curvature of the distal portion (24) can be about 2.3 mm. As to particular embodiments, the distal portion (24) can have the smallest radius of curvature or average radius of curvature of all four portions (23)(24)(25)(26).

The outer arcuate portion (26) can be curved to generally follow the curve of the antihelix (8) and/or the rear of the cavum concha (13). Following, the outer arcuate portion (26) can be convexly arcuate, and can have one or more radii of curvature, which as an illustrative example, can be in a range of between about 6.3 mm and about 15.1 mm. As but one illustrative example, the radius of curvature or the average radius of curvature of the outer arcuate portion (26) can be about 10.4 mm. As to particular embodiments, the outer arcuate portion (26) can have the largest radius of curvature or average radius of curvature of all four portions (23)(24)(25)(26).

The inner arcuate portion (25) can be concavely arcuate, and can have one or more radii of curvature which, as to particular embodiments, can be lesser than the radius of curvature or average radius of curvature of the outer arcuate portion (26). As an illustrative example, the inner arcuate portion (25) can have one or more radii of curvature in a range of between about 3.8 mm and about 10.5 mm. As but one illustrative example, the radius of curvature or the average radius of curvature of the inner arcuate portion (25) can be about 6.4 mm.

As to particular embodiments, the radius of curvature or the average radius of curvature of the inner arcuate portion (25) can be similar to the radius of curvature or average radius of curvature of the proximal portion (23).

Again, with primary reference to Figures 1, following disposition within the concha (9), the portions (23)(24)(25)(26) of the retaining body (17) can be moved to align with one or more of the antihelix (8), antitragus (16), or tragus (15) to provide stable and/or comfortable retention of the retaining body (17) within the ear (3) of the wearer (4).

The retaining body (17) can be fabricated, formed, or molded from a wide variety of elastic or rigid plastics having a suitable hardness (or softness) including, but not necessarily limited to, silicone, acrylic, nylon, acrylonitrile butadiene styrene, polylactic acid, polybenzimidzole, polycarbonate, polyether sulfone, polyethylene, urethane, other like materials, or combinations thereof. As to particular embodiments, the retaining body (17) can comprise an elastically flexible and/or compressible material whereby the retaining body (17) may flex and/or compress slightly when inserted into the concha (9), and it may remain therein via a friction and/or interference fit as the retaining body (17) characteristically tries to unflex and/or expand from the flexed and/or compressed state.

Again, referring primarily to Figures 3 through 4, particular embodiments of the earpiece (2) can further include a projection element (18) outwardly extending from the retaining body inner face (21) to terminate in a projection element end (27). The projection element (18) can project into the ear canal opening (10) to dispose the projection element end (27) within the ear canal (11) when the earpiece (2) is worn by a wearer (4). The retaining body (17) can function to retain the projection element (18) within the ear canal opening (3). The projection element (18) can be configured as a substantially straight (or linear) solid or tubular elongate member having a length and cross-section sufficient for disposition within the ear canal opening (3) and the portion of the ear canal (11) adjacent the ear canal opening (10). As to particular embodiments, the length of the projection element (13) can be in a range of between about 4 millimeters and about 8 millimeters. As but one illustrative example, the projection element length can be about 7.7 millimeters. As to particular embodiments, the projection element (18) can have a circular, oval, square, rectangular, or polygonal cross-sectional configuration, but need not be limited thereto.

The projection element (18) can outwardly extend from a location on the retaining body inner face (21) proximate the proximal portion (23) which aligns the projection element (18) with the ear canal opening (10) for disposition therein concurrent with disposition of the retaining body (17) within the concha (9) when the earpiece (2) is worn by a wearer (4). As to particular embodiments, the projection element (18) can be integrally formed with the retaining body inner face (21) such that the projection element (18) and the retaining body (17) can be formed as a one-piece construct, a monolithic construct, or a unified whole at a location which aligns the projection element (18) with the ear canal opening (10) for disposition therein concurrent with disposition of the retaining body (17) within the concha (9) when the earpiece (2) is worn by a wearer (4).

The projection element (17) can be fabricated, formed, or molded from a wide variety of elastic or rigid plastics having a suitable hardness (or softness) including, but not necessarily limited to, silicone, acrylic, nylon, acrylonitrile butadiene styrene, polylactic acid, polybenzimidzole, polycarbonate, polyether sulfone, polyethylene, urethane, other like materials, or combinations thereof. As to particular embodiments, the projection element (18) can comprise the same material(s) as the retaining body (17).

Again, with primary reference to Figures 3 through 4, the earpiece (2) can, but need not necessarily, further include an earpiece tip (19) configured to dispose about the projection element (18), whereby the earpiece tip (19) can conform to, and in particular embodiments sealably engage with, the ear canal opening (10) or ear canal (11) concurrent with disposition of the retaining body (17) within the concha (9) when the earpiece (2) is worn by a wearer (4). The earpiece tip (19) can comprise any one or a combination of pliant materials which can elastically deform upon engagement with the ear canal opening (10) or ear canal (11) and return toward its original configuration upon disengagement with the ear canal opening (10) or ear canal (11), such as silicone, foam, foam rubber, hydrogel, or the like. As to particular embodiments, the earpiece tip (19) can include a continuously smooth external surface. The earpiece tip (19) can be integrated with the projection element (18) as a one-piece construct, or the earpiece tip (19) can be configured to removably or detachably affix to the projection element (18) such that the earpiece tip (19) can be exchangeable. Regarding the latter configuration, for affixing or coupling, the earpiece tip (19) can include a tip passage (28) axially disposed therein and configured to insertingly receive the projection element (18) to dispose the earpiece tip (19) about the projection element (18). As to particular embodiments, a plurality of earpiece tips (19) can be adapted to or configure to couple with the projection element (18), whereby the plurality of earpiece tips (19) can vary in design, dimension, stiffness, or other structural characteristic allowing the wearer (4) of the earpiece (2) to select the most appropriate variant in order to achieve a particular level of engagement with the ear canal opening (10) or the ear canal (11).

Again, referring primarily to Figures 1 and 3 through 4, as to particular embodiments, the earpiece (2) can function as a passive earplug useful for occluding the ear canal opening (10) to block, whether partially or completely, external sound or noise. In this context, the passive earplug may not include acoustic features because it does not provide sound to the ear (3) of the wearer (4). In other embodiments, the earpiece (2) may include a sound passage communicating between the retaining body outer face (20) and the earpiece tip (19) to allow sound to pass to the ear canal (11).

Now, with primary reference to Figures 1 and 3 through 6F, a faceplate (1) can be configured to cover the retaining body outer face (20) of the retaining body (17). The faceplate (1) can have a faceplate inner face (29) opposite a faceplate outer face (30) extending to a faceplate periphery (31). In particular embodiments, the retaining body (17) and the faceplate (1) can include one or more pairs of releasably engageable mateable parts (32, 33) to removably affix the faceplate (1) to the retaining body (17). As an illustrative example, the releasably engageable mateable parts (32, 33) can comprise a faceplate plug (34) extending from the faceplate inner face (29) and a retaining body aperture (35) defined by an aperture wall (36) disposed in the retaining body (17) open to the retainer body outer face (20) and the retaining body inner face (21). The faceplate plug (34) can be configured to insert into said retainer body aperture (35) to engage said aperture wall (36) to removably affix the faceplate (11) to the retaining body (17). In particular embodiments, the faceplate plug (34) can terminate in a radially extending annular shoulder (37). The radially extending annular shoulder (37) can pass through the retaining body aperture (35) to engage the retaining body inner face (21) to removably affix the faceplate (1) to the retaining body (17) of the earpiece (2).

Again, with primary reference to Figures 1 and 3 through 6F, as a further illustrative example, the releasably engageable mateable parts (32, 33) can comprise a faceplate post (38) extending from the faceplate inner face (29) and a retaining body closed end aperture (39) disposed in and extending inward of the retaining body outer face (20). The faceplate post (38) can be configured to insert into the retaining body closed end aperture (39) to removably affix the faceplate (11) to the retaining body (17) of the earpiece (2). In particular embodiments, the faceplate post (38) can terminate in a barb (40) extending circumferentially around the faceplate post (38).

Again, with primary reference to Figures 1 and 3 through 6F, in particular embodiments, the releasably engageable mateable parts (32, 33) can include a faceplate plug (34) extending from the faceplate inner face (29) of the faceplate (1) and a retaining body aperture (35) defined by an aperture wall (36) disposed in or about the distal portion (24) of the retaining body (17) and open between the retaining body outer face (20) and the retaining body inner face (21), and further include a faceplate post (38) extending from the faceplate inner face (29) and a retaining body closed end aperture (39) disposed in or about the retaining body proximal portion (23) and extending inward of the retaining body outer face (20). The faceplate plug (34) can be configured to insert into the retaining body aperture (35) to engage the aperture wall (36) to removably affix the faceplate (1) to the retaining body (17) and the faceplate post (38) configured to insert into the retaining body closed end aperture (39) to removably affix the faceplate (1) to the retaining body (17). The face plate plug (34) can further include the radially extending annular shoulder (37), as above described, and the face plate post (38) can further include the barb (40), as above described. The above illustrative examples of releasably engageable mateable parts (32, 33) are not intended to preclude the use of releasably engageable mateable parts (32, 33) differently configured but suitable for releasable retention of the faceplate (1) to the retaining body (17).

Now, with primary reference to Figures 1 and 3 through 6F, the faceplate inner face (29) and the faceplate outer face (30) can extend to a faceplate periphery (31). In the illustrative examples, the contours of the faceplate periphery (31) substantially match the contours of the retaining body periphery (22), thereby the faceplate (1) covers the entirety, or substantially the entirety, of the retaining body outer face (20). As these embodiments, the faceplate (1) can comprise a pair of faceplates (1L, 1R), wherein one of the pair of faceplates (1L) is configured to removably affix to the retaining body outer surface (20) of the retaining body (17) configured to dispose within the concha (9) of a left ear (3L) of the wearer (4), and wherein the other one of said pair of faceplates (1R) is configured to removably affix to the retaining body outer surface (20) of the retaining body (17) configured to dispose within the concha (9) of a right ear (3R) of the wearer (4). However, the illustrative embodiment is not intended to obviate embodiments of the faceplate (1) that when affixed to the retaining body (17) do not cover the entirety of the retaining body outer face (20). For example the, faceplate periphery (31) may define faceplate (1) that only covers the proximal portion (23) of the retaining body (17), such as circular faceplate periphery or a polygonal faceplate periphery, or the faceplate periphery (31) may define a faceplate (1) that only covers the distal portion (24) of the retaining body (17), such as circular faceplate periphery or a polygonal faceplate periphery, or the faceplate (1) may comprise a plurality of faceplate components interconnected with open areas between the faceplate components allowing the portions of the retaining body outer face (20) to be viewed through the open areas in the faceplate (1).

Embodiments of the faceplate (1) can be fabricated, formed, or molded from a wide variety of materials, including, but not necessarily limited, one or more of: metals, such as, copper, iron, aluminum, zinc, gold, silver, platinum, titanium, palladium, osmium, and combinations thereof; stones or gems, such as, diamond, pearl, ruby, sapphire, emerald, catseye, alexandrite, amethyst, topaz, tourmaline, aquamarine; ceramic materials, such as clay, brick, glass, tile, cement; plastics, such as, silicone, acrylic, nylon, acrylonitrile butadiene styrene, polylactic acid, polybenzimidzole, polycarbonate, polyether sulfone, polyethylene, urethane; wood; textiles; and combinations thereof.

In particular embodiments, the material used to fabricate, form, or mold the faceplate (1) can affect the acoustic properties of the sound conveyed to the ear canal (11). There can be a difference in acoustic properties of a sound conveyed to the ear canal (11) between faceplates (1) made from different materials. The material used to fabricate, form, or mold the faceplate (1) can be selected to attenuate or amplify all sound frequencies, or attenuate or amplify only particular sound frequencies. Understandably, the acoustic properties can be altered by the selection of the material used to fabricate, form, or mold the faceplate (1).

Now, with primary reference to Figures 1 and 3 through 6F, the faceplate (1) can comprise a plurality of faceplates (1) interchangeably removably affixable with a retaining body outer surface (20). In particular embodiments, each of the plurality of faceplates (1) can have a faceplate outer face (30) having the same visual appearance. In other particular embodiments, each of said plurality of faceplates (1) can have a faceplate outer face (30) having different visual appearance(s). In particular embodiments, the faceplate outer face (30) can be generally flat which affords a surface on which a wide variety of visual elements can be applied. As illustrative examples, the visual elements can include one or more of: a color, a text, an image, artwork, logos, trademarks, symbols, characters, advertisements. or other visual elements that create a three-dimensional effect, adding depth and visual intrigue to the faceplate outer face (30) or to evoke emotions. While in other embodiments the faceplate outer face (30) can comprise an uneven surface having any of a wide and numerous variations in tactile or visual textures that create three dimensional effect which may include, as examples: roughness, rubberiness, bumpiness, stickiness, furriness, prickly, hammered, sculptured or mimic the feel of materials such as glass, wood, concrete, steel, or fabric, or provide reflective, mirrored, or metallic finishes. These illustrative examples are not intended to be comprehensive or to exhaust the possible variations in design of the faceplate (1), but rather are intended to provide sufficient examples to broadly encompass the purpose of the faceplate (1) which allows the wearer (4) to customize the look and feel of the earpiece (2).

Now, with reference primarily to Figures 2 and 3, a method of making an earpiece (2), can include one or more of: configuring a retaining body (17) to dispose within the concha (9) of an ear (3) of a wearer (4), wherein the retaining body (17) comprises a retaining body outer face (20) opposite a retaining body inner face (21) extending to a retaining body periphery (22), wherein the retaining body inner face (21) includes a projection element (18) configured to project into an ear canal opening (10) of the wearer (4) upon disposing the retaining body (17) within the concha (9), wherein the projection element (18) is adapted to retain an earpiece tip (19) configured to engage an ear canal (11) of the wearer (4); and configuring a faceplate (1) to cover said retaining body outer face (20) of the retaining body (17), wherein the retaining body (17) and said faceplate (1) include releasably engageable mateable parts (32, 33) to removably affix the faceplate (1) to the retaining body (17) of the earpiece (2).

Now, with primary reference to Figures 1 and 2, a method of using an earpiece (2) can include one or more of: disposing a retaining body (17) within the concha (9) of an ear (3) of a wearer (4), wherein the retaining body having a retaining body outer face (20) opposite a retaining body inner face (21) extending to a retaining body periphery (22), wherein the retaining body inner face includes a projection element (18) configured to project into an ear canal opening (10) of the wearer (4) upon disposing the retaining body (17) within said concha (9), wherein the projection element (18) adapted to retain an earpiece tip (19) configured to engage the ear canal (11) of the wearer (4); and releasably engaging mateable parts (32, 33) of a faceplate (1) and the retaining body (17) to removably affix the faceplate (1) to said retaining body (17) of the earpiece (2), wherein the faceplate (1) covers in part or in whole the retaining body outer face (20) of the retaining body (17),

As can be easily understood from the foregoing, the basic concepts of the present invention may be embodied in a variety of ways. The invention involves numerous and varied embodiments of a faceplate (1) and an earpiece (2) including a removably affixed faceplate (1) and methods for making and using the faceplate (1), the earpiece (2) and the faceplate (1) affixed to the earpiece (2).

As such, the particular embodiments or elements of the invention disclosed by the description or shown in the figures or tables accompanying this application are not intended to be limiting, but rather exemplary of the numerous and varied embodiments generically encompassed by the invention or equivalents encompassed with respect to any particular element thereof. In addition, the specific description of a single embodiment or element of the invention may not explicitly describe all embodiments or elements possible; many alternatives are implicitly disclosed by the description and figures.

It should be understood that each element of an apparatus or each step of a method may be described by an apparatus term or method term. Such terms can be substituted where desired to make explicit the implicitly broad coverage to which this invention is entitled. As but one example, it should be understood that all steps of a method may be disclosed as an action, a means for taking that action, or as an element which causes that action. Similarly, each element of an apparatus may be disclosed as the physical element or the action which that physical element facilitates. As but one example, the disclosure of "a projection" should be understood to encompass disclosure of the act of "projecting" -- whether explicitly discussed or not -- and, conversely, were there effectively disclosure of the act of "projecting", such a disclosure should be understood to encompass disclosure of "projection" and even a "means for projecting." Such alternative terms for each element or step are to be understood to be explicitly included in the description.

In addition, as to each term used it should be understood that unless its utilization in this application is inconsistent with such interpretation, common dictionary definitions should be understood to be included in the description for each term as contained in the Random House Webster's Unabridged Dictionary, second edition, each definition hereby incorporated by reference.

Moreover, for the purposes of the present invention, the term "a" or "an" entity refers to one or more of that entity; for example, "a protrusion" refers to one or more of those protrusions. As such, the terms "a" or "an", "one or more" and "at least one" can be

As such, the particular embodiments or elements of the invention disclosed by the description or shown in the figures or tables accompanying this application are not intended to be limiting, but rather exemplary of the numerous and varied embodiments generically encompassed by the invention or equivalents encompassed with respect to any particular element thereof. **In** addition, the specific description of a single embodiment or element of the invention may not explicitly describe all embodiments or elements possible; many alternatives are implicitly disclosed by the description and figures.

All numeric values herein are assumed to be modified by the term "about", whether or not explicitly indicated. For the purposes of the present invention, ranges may be expressed as from "about" one particular value to "about" another particular value. When such a range is expressed, another embodiment includes from the one particular value to the other particular value. The recitation of numerical ranges by endpoints includes all the numeric values subsumed within that range. A numerical range of one to five includes for example the numeric values 1, 1.5, 2, 2.75, 3, 3.80, 4, 5, and so forth. It will be further understood that the endpoints of each of the ranges are significant both in relation to the other endpoint, and independently of the other endpoint. When a value is expressed as an approximation by use of the antecedent "about," it will be understood that the particular value forms another embodiment. The term "about" generally refers to a range of numeric values that one of skill in the art would consider equivalent to the recited numeric value or having the same function or result. Similarly, the antecedent "substantially" means largely, but not wholly, the same form, manner or degree and the particular element will have a range of configurations as a person of ordinary skill in the art would consider as having the same function or result. When a particular element is expressed as an approximation by use of the antecedent "substantially," it will be understood that the particular element forms another embodiment.

Further, for the purposes of the present invention, the term "coupled" or derivatives thereof can mean indirectly coupled, coupled, directly coupled, connected, directly connected, or integrated with, depending upon the embodiment.

Additionally, for the purposes of the present invention, the term "integrated" when referring to two or more components means that the components (i) can be united to provide a one-piece construct, a monolithic construct, or a unified whole, or (ii) can be formed as a one-piece construct, a monolithic construct, or a unified whole. Said another way, the components can be integrally formed, meaning connected together so as to make up a single complete piece or unit, or so as to work together as a single complete piece or unit, and so as to be incapable of being easily dismantled without destroying the integrity of the piece or unit.

Thus, the applicant(s) should be understood to claim at least: i) each of the faceplate and earpiece herein disclosed and described, ii) the related methods disclosed and described, iii) similar, equivalent, and even implicit variations of each of these devices and methods, iv) those alternative embodiments which accomplish each of the functions shown, disclosed, or described, v) those alternative designs and methods which accomplish each of the functions shown as are implicit to accomplish that which is disclosed and described, vi) each feature, component, and step shown as separate and independent inventions, vii) the applications enhanced by the various systems or components disclosed, viii) the resulting products produced by such systems or components, ix) methods and apparatuses substantially as described hereinbefore and with reference to any of the accompanying examples, x) the various combinations and permutations of each of the previous elements disclosed.

The background section of this patent application, if any, provides a statement of the field of endeavor to which the invention pertains. This section may also incorporate or contain paraphrasing of certain United States patents, patent applications, publications, or subject matter of the claimed invention useful in relating information, problems, or concerns about the state of technology to which the invention is drawn toward. It is not intended that any United States patent, patent application, publication, statement or other information cited or incorporated herein be interpreted, construed or deemed to be admitted as prior art with respect to the invention.

The claims set forth in this specification, if any, are hereby incorporated by reference as part of this description of the invention, and the applicant expressly reserves the right to use all of or a portion of such incorporated content of such claims as additional description to support any of or all of the claims or any element or component thereof, and the applicant further expressly reserves the right to move any portion of or all of the incorporated content of such claims or any element or component thereof from the description into the claims or vice-versa as necessary to define the matter for which protection is sought by this application or by any subsequent application or continuation, division, or continuation-in-part application thereof, or to obtain any benefit of, reduction in fees pursuant to, or to comply with the patent laws, rules, or regulations of any country or treaty, and such content incorporated by reference shall survive during the entire pendency of this application including any subsequent continuation, division, or continuation-in-part application thereof or any reissue or extension thereon. The elements following an open transitional phrase such as "comprising" may in the alternative be claimed with a closed transitional phrase such as "consisting essentially of" or "consisting of" whether or not explicitly indicated the description portion of the specification.

Additionally, the claims set forth in this specification, if any, are further intended to describe the metes and bounds of a limited number of the preferred embodiments of the invention and are not to be construed as the broadest embodiment of the invention or a complete listing of embodiments of the invention that may be claimed. The applicant does not waive any right to develop further claims based upon the description set forth above as a part of any continuation, division, or continuation-in-part, or similar application.
In the following clauses preferred embodiments of the application are described:
1. An earpiece, comprising:
   a retaining body configured to dispose within the concha of an ear of a wearer,
      said retaining body having a retaining body outer face opposite a retaining body inner face extending to a retaining body periphery,
      said retaining body inner face including a projection element configured to project into an ear canal opening of said wearer upon disposing said retaining body within said concha,
      said projection element adapted to retain an earpiece tip configured to engage an ear canal wall of said wearer; and
   a faceplate configured to cover said retaining body outer face of said retaining body,
      said retaining body and said faceplate including releasably engageable mateable parts to removably affix said faceplate to said retaining body.
2. The earpiece of clause 1, wherein said retaining body configured to dispose within said concha of a left ear of said wearer.
3. The earpiece of clause 1, wherein said retaining body configured to dispose within said concha of a right ear of said wearer.
4. The earpiece of clause 1, wherein said retaining body comprise a pair of retaining bodies, wherein one of said pair of retaining bodies configured to dispose within said concha of a left ear of said wearer, wherein one of said pair of retaining bodies configured to dispose within said concha of a right ear of said wearer.
5. The earpiece of clause 2, wherein said faceplate configured to removably affix to said outer surface of said retaining body configured to dispose within said concha of a left ear of said wearer.
6. The earpiece of clause 3, wherein said faceplate configured to removably affix to said outer surface of said retaining body configured to dispose within said concha of a right ear of said wearer.
7. The earpiece of clause 4, wherein said faceplate comprises a pair of faceplates, wherein one of said pair of faceplates configured to removably affix to said outer surface of said retaining body configured to dispose within said concha of a left ear of said wearer, wherein one of said pair of faceplates configured to removably affix to said outer surface of said retaining body configured to dispose within said concha of a right ear of said wearer.
8. The earpiece of clause 1, wherein said releasably engageable mateable parts comprise a faceplate plug extending from said faceplate and a retaining body aperture defined by an aperture wall, said retaining body aperture open between said retainer body outer face and said retaining body inner face, said faceplate plug configured to insert into said retainer body aperture to engage said aperture wall to removably affix said faceplate to said retaining body.
9. The earpiece of clause 8, wherein said faceplate plug comprise a cylindrical plug terminating in a radially extending annular shoulder, said radially extending annular shoulder passes through said retaining body aperture to engage said retaining body inner face.
10. The earpiece of clause 1, wherein said releasably engageable mateable parts comprise a faceplate post extending from said faceplate and a retaining body closed end aperture extending inward of said retaining body outer face, said faceplate post configured to insert into said retaining body closed end aperture to removably affix said faceplate to said retaining body.
11. The earpiece of clause 10, wherein said faceplate post terminates in a barb extending circumferentially around said faceplate post.
12. The earpiece of clause 1, wherein said releasably engageable mateable parts comprise:
   a faceplate plug extending from said faceplate and a retaining body aperture defined by an aperture wall, said retaining body aperture open between said retainer body outer face and said retaining body inner face, said faceplate plug configured to insert into said retaining body aperture to engage said aperture wall to removably affix said faceplate to said retaining body; and
   a faceplate post extending from said faceplate and a retaining body closed end aperture extending inward of said retaining body outer face, said faceplate post configured to insert into said retaining body closed end aperture to removably affix said faceplate to said retaining body.
13. The earpiece of clause 12, wherein said faceplate plug comprise a cylindrical plug terminating in a radially extending annular shoulder, said radially extending annular shoulder passes through said retaining body aperture to engage said retaining body inner face, and wherein said faceplate post terminates in a barb circumferentially around said faceplate post, said barb passes into said retaining body closed end aperture to removably affix said faceplate to said retaining body.
14. The earpiece of clause 1, wherein said faceplate has a faceplate inner face opposite a faceplate outer face extending to a faceplate periphery, wherein said releasably engageable mateable parts of said faceplate extend from said faceplate inner surface.
15. The earpiece of clause 14, wherein said faceplate periphery configured to generally match said retaining body periphery.
16. The earpiece of clause 14, wherein said faceplate outer surface generally flat.
17. The earpiece of clause 1, wherein said faceplate comprises a plurality of faceplates interchangeably removably affixable with said retaining body outer surface.
18. The earpiece of clause 17, wherein each of said plurality of faceplates having a faceplate outer surface having the same visual appearance.
19. The earpiece of clause 17, wherein each of said plurality of faceplates having a faceplate outer surface having different visual appearance.
20. The earpiece of claim 17, wherein said visual appearance of said faceplate outer surface comprises one or more of a color, a text, an image, a two dimensional design, a three dimensional structure.
21. The earpiece of clause 1, wherein said projection element integrally formed with said retaining body.
22. The earpiece of clause 1, wherein said projection element and said retaining body formed as a one-piece construct.
23. The earpiece of clause 1, wherein said earpiece tip engages with said ear canal wall when said earpiece is worn by said wearer.
24. The earpiece of clause 23, wherein said earpiece tip sealably engages with said ear canal wall when said earpiece is worn by said wearer.
25. A faceplate, comprising:
   a faceplate configured to removably affix to an outer face of a retaining body of said earpiece, said retaining body configured to dispose within the concha of an ear of a wearer.
26. The earpiece of clause 25, wherein,
   said retaining body having a retaining body inner face opposite said retaining body outer face extending to a retaining body periphery,
   said retaining body inner face including a projection element configured to project into an ear canal opening of said wearer upon disposing said retaining body within said concha,
   said projection element adapted to retain an earpiece tip configured to engage an ear canal wall of said wearer; and
   said retaining body and said faceplate including releasably engageable mateable parts to removably affix said faceplate to said retaining body.
27. A method of making an earpiece, comprising:
   configuring a retaining body to dispose within the concha of an ear of a wearer,
      said retaining body having a retaining body outer face opposite a retaining body inner face extending to a retaining body periphery,
      said retaining body inner face including a projection element configured to project into an ear canal opening of said wearer upon disposing said retaining body within said concha,
      said projection element adapted to retain an earpiece tip configured to engage an ear canal wall of said wearer; and
   configuring a faceplate to cover said retaining body outer face of said retaining body,
      said retaining body and said faceplate including releasably engageable mateable parts to removably affix said faceplate to said retaining body.
28. A method of using an earpiece, comprising:
   disposing a retaining body within the concha of an ear of a wearer,
      said retaining body having a retaining body outer face opposite a retaining body inner face extending to a retaining body periphery,
      said retaining body inner face including a projection element configured to project into an ear canal opening of said wearer upon disposing said retaining body within said concha,
      said projection element adapted to retain an earpiece tip configured to engage an ear canal wall of said wearer; and
   releasably engaging mateable parts of a faceplate and said retaining body to removably affix said faceplate to said retaining body,
      said faceplate configured to cover said retaining body outer face of said retaining body.

## Claims

1. An earpiece, comprising:
a retaining body configured to dispose within the concha of an ear of a wearer,
said retaining body having a retaining body outer face opposite a retaining body inner face extending to a retaining body periphery,
said retaining body inner face including a projection element configured to project into an ear canal opening of said wearer upon disposing said retaining body within said concha,
said projection element adapted to retain an earpiece tip configured to engage an ear canal wall of said wearer; and
a faceplate configured to cover said retaining body outer face of said retaining body,
said retaining body and said faceplate including releasably engageable mateable parts to removably affix said faceplate to said retaining body.

2. The earpiece of claim 1, wherein said retaining body configured to dispose within said concha of a left ear of said wearer, preferably wherein said faceplate configured to removably affix to said outer surface of said retaining body configured to dispose within said concha of a left ear of said wearer.

3. The earpiece of claim 1, wherein said retaining body configured to dispose within said concha of a right ear of said wearer, preferably wherein said faceplate configured to removably affix to said outer surface of said retaining body configured to dispose within said concha of a right ear of said wearer.

4. The earpiece of claim 1, wherein said retaining body comprise a pair of retaining bodies, wherein one of said pair of retaining bodies configured to dispose within said concha of a left ear of said wearer, wherein one of said pair of retaining bodies configured to dispose within said concha of a right ear of said wearer, preferably wherein said faceplate comprises a pair of faceplates, wherein one of said pair of faceplates configured to removably affix to said outer surface of said retaining body configured to dispose within said concha of a left ear of said wearer, wherein one of said pair of faceplates configured to removably affix to said outer surface of said retaining body configured to dispose within said concha of a right ear of said wearer.

5. The earpiece of claim 1, wherein said releasably engageable mateable parts comprise a faceplate plug extending from said faceplate and a retaining body aperture defined by an aperture wall, said retaining body aperture open between said retainer body outer face and said retaining body inner face, said faceplate plug configured to insert into said retainer body aperture to engage said aperture wall to removably affix said faceplate to said retaining body, preferably wherein said faceplate plug comprise a cylindrical plug terminating in a radially extending annular shoulder, said radially extending annular shoulder passes through said retaining body aperture to engage said retaining body inner face.

6. The earpiece of claim 1, wherein said releasably engageable mateable parts comprise a faceplate post extending from said faceplate and a retaining body closed end aperture extending inward of said retaining body outer face, said faceplate post configured to insert into said retaining body closed end aperture to removably affix said faceplate to said retaining body, preferably wherein said faceplate post terminates in a barb extending circumferentially around said faceplate post.

7. The earpiece of claim 1, wherein said releasably engageable mateable parts comprise:
a faceplate plug extending from said faceplate and a retaining body aperture defined by an aperture wall, said retaining body aperture open between said retainer body outer face and said retaining body inner face, said faceplate plug configured to insert into said retaining body aperture to engage said aperture wall to removably affix said faceplate to said retaining body; and
a faceplate post extending from said faceplate and a retaining body closed end aperture extending inward of said retaining body outer face, said faceplate post configured to insert into said retaining body closed end aperture to removably affix said faceplate to said retaining body, preferably wherein said faceplate plug comprise a cylindrical plug terminating in a radially extending annular shoulder, said radially extending annular shoulder passes through said retaining body aperture to engage said retaining body inner face, and wherein said faceplate post terminates in a barb circumferentially around said faceplate post, said barb passes into said retaining body closed end aperture to removably affix said faceplate to said retaining body.

8. The earpiece of claim 1, wherein said faceplate has a faceplate inner face opposite a faceplate outer face extending to a faceplate periphery, wherein said releasably engageable mateable parts of said faceplate extend from said faceplate inner surface.

9. The earpiece of claim 8, wherein said faceplate periphery configured to generally match said retaining body periphery, or wherein said faceplate outer surface generally flat.

10. The earpiece of claim 1, wherein said faceplate comprises a plurality of faceplates interchangeably removably affixable with said retaining body outer surface.

11. The earpiece of claim 10, wherein each of said plurality of faceplates having a faceplate outer surface having the same visual appearance, or
wherein each of said plurality of faceplates having a faceplate outer surface having different visual appearance, or
wherein said visual appearance of said faceplate outer surface comprises one or more of a color, a text, an image, a two dimensional design, a three dimensional structure.

12. The earpiece of claim 1, wherein said projection element integrally formed with said retaining body, or wherein said projection element and said retaining body formed as a one-piece construct.

13. The earpiece of claim 1, wherein said earpiece tip engages with said ear canal wall when said earpiece is worn by said wearer, preferably wherein said earpiece tip sealably engages with said ear canal wall when said earpiece is worn by said wearer.

14. A faceplate, comprising:
a faceplate configured to removably affix to an outer face of a retaining body of said earpiece, said retaining body configured to dispose within the concha of an ear of a wearer, preferably wherein,
said retaining body having a retaining body inner face opposite said retaining body outer face extending to a retaining body periphery,
said retaining body inner face including a projection element configured to project into an ear canal opening of said wearer upon disposing said retaining body within said concha,
said projection element adapted to retain an earpiece tip configured to engage an ear canal wall of said wearer; and
said retaining body and said faceplate including releasably engageable mateable parts to removably affix said faceplate to said retaining body.

15. A method of making an earpiece, comprising:
configuring a retaining body to dispose within the concha of an ear of a wearer,
said retaining body having a retaining body outer face opposite a retaining body inner face extending to a retaining body periphery,
said retaining body inner face including a projection element configured to project into an ear canal opening of said wearer upon disposing said retaining body within said concha,
said projection element adapted to retain an earpiece tip configured to engage an ear canal wall of said wearer; and
configuring a faceplate to cover said retaining body outer face of said retaining body,
said retaining body and said faceplate including releasably engageable mateable parts to removably affix said faceplate to said retaining body.

16. A method of using an earpiece, comprising:
disposing a retaining body within the concha of an ear of a wearer,
said retaining body having a retaining body outer face opposite a retaining body inner face extending to a retaining body periphery,
said retaining body inner face including a projection element configured to project into an ear canal opening of said wearer upon disposing said retaining body within said concha,
said projection element adapted to retain an earpiece tip configured to engage an ear canal wall of said wearer; and
releasably engaging mateable parts of a faceplate and said retaining body to removably affix said faceplate to said retaining body,
said faceplate configured to cover said retaining body outer face of said retaining body.
